(19) Europäisches Patentamt

European Patent Office

Office européen des brevets

(11) **EP 4 691 590 A1**

(12) **EUROPEAN PATENT APPLICATION**
published in accordance with Art. 153(4) EPC

(43) Date of publication:
**11.02.2026 Bulletin 2026/07**

(21) Application number: **24850250.2**

(22) Date of filing: **25.06.2024**

(51) International Patent Classification (IPC):
**B01D 3/32** (2006.01)     **B01D 3/22** (2006.01)
**B01D 3/42** (2006.01)     **C07C 51/44** (2006.01)
**C07C 67/54** (2006.01)     **C07C 7/04** (2006.01)
**C07C 57/04** (2006.01)     **C07C 69/54** (2006.01)
**C07C 11/167** (2006.01)

(52) Cooperative Patent Classification (CPC):
**B01D 3/22; B01D 3/32; B01D 3/42; C07C 7/04;
C07C 11/167; C07C 51/44; C07C 57/04;
C07C 67/54; C07C 69/54**

(86) International application number:
**PCT/KR2024/008755**

(87) International publication number:
**WO 2025/063436 (27.03.2025 Gazette 2025/13)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **19.09.2023   KR 20230124667**

(71) Applicant: **LG Chem, Ltd.
Seoul 07336 (KR)**

(72) Inventors:
• **OH, Jai June
  Yuseong-gu
  Daejeon 34122 (KR)**
• **JANG, Kyung Soo
  Yuseong-gu
  Daejeon 34122 (KR)**
• **LEE, Sung Kyu
  Yuseong-gu
  Daejeon 34122 (KR)**

(74) Representative: **Goddar, Heinz J.
Boehmert & Boehmert
Anwaltspartnerschaft mbB
Pettenkoferstrasse 22
80336 München (DE)**

(54) **REACTIVE MONOMER SEPARATION APPARATUS**

(57)     The present disclosure relates to a separation device, and more specifically, to a separation device in which a structure of a feed supply unit is designed so that feed can be uniformly distributed inside a column, thereby improving column efficiency.

FIG. 3

EP 4 691 590 A1

## Description

Cross Reference to Related Applications

This application claims priority to and the benefit of Korean Patent Application No. 10-2023-0124667, filed on September 19, 2023, which is hereby incorporated by reference in their entirety.

Technical Field

The present disclosure relates to a separation device, and more specifically, to a reactive monomer separation device in which a structure of a feed supply unit is designed so that feed can be uniformly distributed inside a column, thereby improving column efficiency.

## Background Art

A dual flow tray is a distillation device designed so that gas and liquid move up and down through a hole and make contact without a downcomer. Specifically, the dual flow tray is used in a case where liquid flow occurs due to weeping liquid in the hole of the tray since the downcomer is not provided, and a substance to be separated is severely polymerized inside the column to form a polymer, and fouling is a concern.

The dual flow tray has the advantage of a large allowable flow rate and can be used to respond to polymer fouling, but has the disadvantage of being lower in efficiency than columns using other types of existing trays due to a channeling phenomenon in which flow occurs without exchange of liquid and vapor. The channeling phenomenon is the main factor that reduces the efficiency of a column, and the larger the diameter of the column, the more likely it is that the channeling phenomenon occurs.

The channeling phenomenon is a phenomenon in which the liquid and gas flow is unevenly distributed due to physical factors such as uneven distribution of feed or tray blockage, and when this channeling phenomenon occurs, the gas-liquid contact area decreases, and as a result, the gas-liquid contact for each stage occurs in a smaller region compared to the design capacity. In addition, since there is no downcomer in the dual flow tray, the liquid redistribution does not occur, and as a result, the overall efficiency of the column decreases, which causes problems such as a decrease in product quality and an increase in energy consumption.

In this way, since the uneven distribution of feed inside the column in the column using the dual flow tray generates the channeling phenomenon, even distribution of feed is one of the factors that determines the efficiency of the column, but in the related art, even distribution of feed within the column is designed without being considered, and the column is operated with low efficiency. Therefore, it is necessary to design a feed supply structure that enables even distribution of feed in the column using the dual flow tray.

## Brief Description

## Technical Problem

The problem to be solved by the present disclosure is to solve the problems mentioned in the Background technology of the above invention, and an object of the present disclosure is to provide a reactive monomer separation device with improved column efficiency by designing a structure of a feed supply unit so that the feed can be uniformly distributed within the column in order to solve the problem mentioned in the technology that is the background of the above disclosure.

However, an object of the present disclosure is not limited to the object mentioned above, and other objects that are not mentioned can be clearly understood by those skilled in the art from the description below.

## Technical Solution

According to an embodiment of the present disclosure, there is provided a reactive monomer separation device including: a cylindrical column; a plurality of dual flow trays provided inside the cylindrical column to partition a plurality of stages; and a feed supply unit provided in one of the plurality of stages to supply a raw material including a liquid reactive monomer.

Moreover, the feed supply unit may include a circular feed transfer pipe spaced apart between a lower dual flow tray and an upper dual flow tray of the stage in which the feed supply unit is provided, and provided along an inner wall of the cylindrical column, a plurality of inner pipes extending in a direction of a central axis from the feed transfer pipe, and a plurality of spray nozzles provided in the plurality of inner pipes, and each of the plurality of inner pipes may be individually provided with a plurality of the spray nozzles.

## Advantageous Effects

According to the reactive monomer separation device of the present disclosure, it is possible to prevent fouling by using the dual flow tray and design the structure of the feed supply unit so that the area of the feed distribution region of the feed stage in the dual flow tray can be maximized to supply raw materials evenly, and thus, it is possible to prevent channeling phenomenon and maximize the gas-liquid contact area to increase the efficiency of the entire column.

More specifically, according to the reactive monomer separation device of the present disclosure, it is possible to improve the structure of the feed supply unit so that the feed can be distributed evenly in the dual flow tray to prevent the channeling phenomenon and improve the separation efficiency of the column, and thus, it is possible to improve the quality of a finally obtained product and also to reduce energy usage.

The effects that can be obtained in the present specification are not limited to the effects mentioned above, and other effects that are not mentioned can be clearly understood by those skilled in the art to which the present disclosure belongs from the description below.

## BRIEF DESCRIPTION OF THE DRAWINGS

FIG. 1 is a cross-sectional view of a reactive monomer separation device according to an embodiment of the present disclosure.

FIG. 2 is a perspective view illustrating a detailed structure of a region A of FIG. 1 according to an embodiment of the present disclosure and illustrating a feed supply unit having a spray nozzle provided at the bottom of a straight inner pipe.

FIG. 3 is a perspective view illustrating the detailed structure of the region A of FIG. 1 according to an embodiment of the present disclosure and illustrating a feed supply unit having a spray nozzle provided at the bottom of a branched inner pipe.

FIG. 4 is a perspective view illustrating the detailed structure of the region A of FIG. 1 according to an embodiment of the present disclosure and illustrating a feed supply unit having a spray nozzle provided at the top of a straight inner pipe.

FIG. 5 is a perspective view illustrating the detailed structure of the region A of FIG. 1 according to an embodiment of the present disclosure and illustrating a feed supply unit having a spray nozzle provided at the top of a branched inner pipe.

FIG. 6 is a perspective view illustrating feed spray when six straight inner pipes are provided as an embodiment of the present disclosure.

FIG. 7 is a top view illustrating a feed spray region B formed in a dual flow tray when six straight inner pipes are provided as an embodiment of the present disclosure.

FIG. 8 is a perspective view illustrating feed spray when two branched inner pipes are provided as an embodiment of the present disclosure.

FIG. 9 is a top view illustrating a feed spray region B formed in a dual flow tray when two branched inner pipes are provided as an embodiment of the present disclosure.

FIG. 10 is a perspective view illustrating feed spray when three branched inner pipes are provided as an embodiment of the present disclosure.

FIG. 11 is a top view illustrating a feed spray region B formed in a dual flow tray when three branched inner pipes are provided as an embodiment of the present disclosure.

FIG. 12 is a perspective view illustrating feed spray when four branched inner pipes are provided as an embodiment of the present disclosure.

FIG. 13 is a top view illustrating a feed spray region B formed in a dual flow tray when four branched inner pipes are provided as an embodiment of the present disclosure.

FIG. 14 is a perspective view illustrating feed spray when only one spray nozzle is provided for each of four inner pipes as one comparative example of the present disclosure.

FIG. 15 is a top view illustrating a feed spray region formed in a dual flow tray when only one spray nozzle is provided for each of four inner pipes as one comparative example of the present disclosure.

FIG. 16 is a side view illustrating a feed spray region B formed when the spray nozzle is provided in a form inclined at a predetermined angle as an embodiment of the present disclosure.

FIG. 17 is a top view illustrating a feed spray region B formed in a dual flow tray when a spray nozzle is provided in two branched inner pipes in a form inclined at a predetermined angle as an embodiment of the present disclosure.

**Detailed Description**

Terms or words used in the description and claims of the present disclosure should not be interpreted as limited to their usual or dictionary meanings, and should be interpreted as meanings and concepts that conform to the technical idea of the present disclosure based on the principle that the inventor can appropriately define the concept of the term in order to explain his own disclosure in the best way.

In relation to the description of the drawings, similar reference numerals may be used for similar or related components.

The singular form of a noun corresponding to an item may include one or more of the items, unless the relevant context clearly indicates otherwise.

In the present disclosure, each of the phrases such as "A or B", "at least one of A and B", "at least one of A or B", "A, B or C", "at least one of A, B and C", and "at least one of A, B, or C" may include any one of the items listed together in the corresponding phrase, or all possible combinations thereof.

The term "and/or" includes a combination of multiple related described components or any one of multiple related described components.

Terms such as "first," "second," or "first" or "second" may be used simply to distinguish the corresponding component from other corresponding components and do not limit the corresponding components in any other aspect (for example, importance or order).

In addition, terms such as "front surface", "back surface", "upper surface", "lower surface", "side surface", "left", "right", "upper portion", and "lower portion" used herein are defined based on the drawings, and the shape and position of each component are not limited by these terms.

Terms such as "include" or "have" are intended to specify the presence of a feature, number, step, operation, component, part, or combination thereof described in the present disclosure, but do not preclude the presence or addition of one or more other features, numbers, steps, operations, components, parts, or combinations thereof.

When a component is said to be "connected", "coupled" "supported", or "contacted" with another component, this includes not only cases where the components are directly connected, coupled, supported, or contacted, but also cases where the components are indirectly connected, coupled, supported, or contacted through a third component.

When a component is said to be "on" another component, this includes not only cases where the component is in contact with the other component, but also cases where another component exists between the two components.

In addition, as used herein, the terms "about" and "substantially" are used to mean at or near the numerical value when a unique manufacturing and material tolerance is presented, and are used to prevent unscrupulous infringers from unfairly utilizing the disclosure in which exact or absolute values are mentioned to help understanding of the present disclosure.

The term "stream" used herein may mean the flow of fluid within a process, and may also mean the fluid itself flowing within a pipe. Specifically, the stream may mean both the fluid itself flowing within a pipe connecting each device and the flow of the fluid. In addition, the fluid may include at least one component of gas, liquid, and solid.

In the present disclosure, a "bottom" of the device means, unless otherwise specified, a point at a height of 95% to 100% downward from the uppermost portion of the device, and may specifically mean the lowest part. Similarly, a "top" of the device means, unless otherwise specified, a point at a height of 0% to 5% downward from the uppermost portion of the device, and may specifically mean the highest part.

In the present disclosure, a "depth of a spray nozzle" means a distance from the inside of a column 100 located most adjacent to a spray nozzle 330, and a "height of the spray nozzle" may mean a height from a lower tray 210a to the feed supply unit 300. In addition, a "tray spacing TS" may mean a height from the lower tray 210a to an upper tray 210b at any stage.

In the present disclosure, a "feed spray region B" may mean the total area where the feed sprayed from the spray nozzle comes into contact with the tray.

Hereinafter, a reactive monomer separation device according to the present disclosure will be described in detail with reference to the drawings.

The reactive monomer separation device according to the present disclosure is for purifying and separating highly reactive monomers such as (meth)acrylic acid, acrylic acid, methyl acrylate, or 1,3-butadiene, and more specifically, may be a reduced pressure distillation column. For example, the reactive monomer such as acrylic acid forms a polymer by a Michael addition reaction at a high temperature of about 100°C or higher, and has a boiling point of about 141°C at normal pressure. When distillation purification is performed at normal pressure, polymer formation occurs at the bottom of the distillation column, making continuous operation of the process impossible. In this case, a reactive monomer purification process should be designed as a reduced pressure process to lower the temperature of the column.

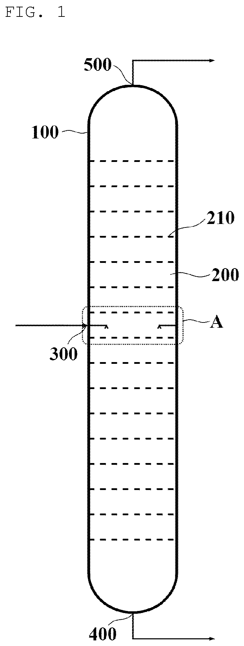

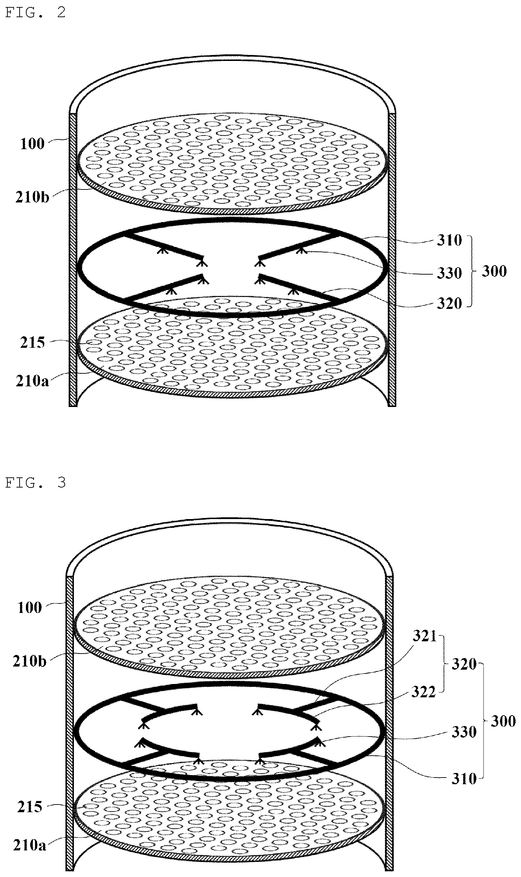

FIG. 1 is a cross-sectional view of a reactive monomer separation device according to an embodiment, and FIGS. 2 to 4 are perspective views illustrating an enlarged view of a region A of FIG. 1, and more specifically, a detailed structural diagram of a feed stage A equipped with a feed supply unit 300.

Referring to FIG. 1, the reactive monomer separation device according to an embodiment of the present disclosure includes a cylindrical column 100.

The cylindrical column 100 can be applied without limitation as long as it is a form generally used in a separation process through gas-liquid contact, and the size of the cylindrical column 100 can be appropriately selected within a range commonly applied in the relevant field, and there is no special limitation. For example, an inner diameter of the cylindrical column 100 may be about 300 mm to 6,000 mm, specifically about 1,500 mm to 6,000 mm, more specifically about 1,800 mm to 4,000 mm, and the overall height of the cylindrical column 100 may be about 2,000 mm to 60,000 mm, specifically about 5,000 mm to 50,000 mm, more specifically about 6,000 mm to 40,000 mm, but is not limited thereto.

In addition, the reactive monomer separation device according to an embodiment of the present disclosure is equipped with a plurality of dual flow trays 210 inside the cylindrical column 100. The reactive monomer, which is the separation target material, is easily polymerized to form a polymer, so there is a high risk of fouling. Therefore, the separation device according to the present disclosure uses the dual flow tray 210 as a tray that divides the stage of the distillation column for gas-liquid separation.

The dual flow tray 210 is a sieve tray without a downcomer, and performs a dual function of allowing both liquid and vapor to pass through a hole 215 of the dual flow tray. In addition, since the dual flow tray does not have a downcomer, a larger tray region is provided, and thus, it has the advantage of having a larger capacity than a general tray type and being easy to install and maintain. However, since the dual flow tray does not have the downcomer, redistribution of the liquid does not occur, and the liquid continuously flows through the hole 215, it is low in efficiency and is susceptible to channeling phenomenon, making it sensitive to the liquid distribution of the feed. In other words, in order to prevent the channeling phenomenon, it is necessary to add a nozzle to evenly distribute the feed, but especially in the reduced pressure distillation column, a manhole and flange must be minimized to maintain the negative pressure inside the column, so it may be difficult to add a subsequent spray nozzle.

In addition, due to the frequent occurrence of pipe clogging due to the polymerization of the reactive monomer which is the raw material, a complex structure device used in a general column, such as a liquid distributor, cannot be used, and the spray nozzle 330 must be used to ensure that the feed is distributed as evenly as possible on the tray. Accordingly, in the present disclosure, the feed supply unit 300 is designed to be supplied by distributing the liquid feed as evenly as possible to prevent the channeling phenomenon occurring in the dual flow tray, thereby improving the column separation efficiency, obtaining high-quality products, and providing a reactive monomer separation device that can reduce energy consumption.

The plurality of dual flow trays 210 are arranged in a vertical direction with respect to the height direction of the cylindrical column 100 as illustrated in FIG. 1, and can be arranged to be spaced apart by a predetermined interval TS. The interior of the cylindrical column 100 can be partitioned into a plurality of stages 200 by the plurality of dual flow trays 210.

The number and size of the dual flow trays 210 are not particularly limited, and can be set based on the number of theoretical stages, or the like inferred from a distillation curve considering the composition of the feed stream. In the above, the "number of theoretical stages" means a virtual region where two phases, such as a gas phase and a liquid phase, are in equilibrium with each other in a separation device, or the number of stages partitioned by the plurality of trays.

According to an embodiment of the present disclosure, the feed supply unit 300 is provided in one of the plurality of stages 200, that is, a predetermined feed stage A, so as to supply a liquid raw material (feed) into the cylindrical column 100. Here, the raw material may include one or more reactive monomers, or a mixture thereof. For example, the reactive monomer may include, but is not limited to, (meth)acrylic acid, acrylic acid, methyl acrylate, 1,3-butadiene, or a mixture thereof.

In addition, the feed supply unit 300 may be spaced apart from and arranged between the lower dual flow tray 210a and the upper dual flow tray 210b in the predetermined feed stage A. The height of the feed supply unit 300 may be determined according to the tray spacing TS, the column inner diameter, the depth of the spray nozzle, a spray angle X, and/or the feed spray direction.

For example, the height of the feed supply unit 300 may be more than 0 mm and less than TS, and more specifically, the height may be 0.25*TS to 0.75*TS. When the feed supply unit 300 is positioned too low, the feed supply unit 300 may be positioned below the feed solution level (liquid level) on the lower dual flow tray 210a. In this case, the solution level can be calculated using a simulation program, or the like. In addition, when the feed supply unit 300 is too low or high, the feed can flow down the inner wall of the column, which may reduce the efficiency of the column.

Referring to FIGS. 2 to 4, in the reactive monomer separation device according to an embodiment of the present disclosure, the feed supply unit 300 includes a feed transfer pipe 310, an inner pipe 320, and the spray nozzle 330.

In an embodiment of the present disclosure, the feed transfer pipe 310 may be provided in a circular shape along the inner wall of the cylindrical column 100. Through the structure described above, interference of a solution descending from the upper tray 210b to the lower tray 210a can be minimized.

In an embodiment of the present disclosure, the inner pipe 320 is provided to extend from the circular feed transfer pipe 310 in the direction of the central axis. More specifically, the length direction of the inner pipe 320 may be provided in a horizontal direction with respect to the dual flow tray 210. In addition, a plurality of the inner pipes 320 may be provided, and for example, the number of the inner pipes may be 2 to 8, specifically, 2 to 7, and more specifically, 2 to 6.

In addition, one or more inner pipes 320 may be provided to be spaced apart from each other at equal intervals. When the plurality of inner pipes 320 are arranged spaced apart from each other at equal intervals, the feed spray regions B can be more uniformly distributed by minimizing an overlapping region in which the feed spray regions overlap each other and a non-spray region.

In an embodiment of the present disclosure, the inner pipe 320 may have a form of a "straight inner pipe" extending in the direction of the central axis from the feed transfer pipe 310 as illustrated in FIG. 2, or a "branched inner pipe" including a connecting pipe 321 in the direction of the central axis extending from the feed transfer pipe 310 and a branch pipe 322 provided at an end of the connecting pipe as illustrated in FIG. 3.

The branch pipe 322 is provided in a vertical direction with respect to the length direction of the connecting pipe 321, and may be provided in a horizontal direction with respect to the dual flow tray 210. In addition, the branch pipe 322 may have a straight or curved shape. For example, when the branch pipe 322 has a curved shape, the branch pipe may be provided in parallel with respect to the feed transfer pipe 310 at a certain interval. In addition, in the plurality of branch pipes 322, the ends of each branch pipe may not be in contact with each other and may be spaced apart at the same interval.

Meanwhile, the length of the inner pipe 320 with respect to the central axis direction may be less than 0.5 times, specifically 0.1 to 0.4 times the inner diameter of the cylindrical column 100.

Specifically, when the inner pipe 320 is a straight inner pipe, a length that allows the inner wall of the cylindrical column and two or more spray nozzles to be spaced apart from each other at a certain interval must be secured. The length of the straight inner pipe may be, for example, less than 0.5 times, specifically 0.1 to 0.45 times, or more specifically 0.2 to 0.4 times the inner diameter of the cylindrical column 100.

Meanwhile, in the case where the inner pipe 320 is the branched inner pipe, since the space of the branch pipe must be secured, it may be preferable that the length of the connecting pipe 321 be shorter than that of the straight inner pipe. For example, the length of the connecting pipe 321 of the branched inner pipe may be 0.4 times or less, specifically 0.1 to 0.35 times, or more specifically 0.1 to 0.3 times the inner diameter of the cylindrical column 100. The longer the length of the connecting pipe 321, the larger the maximum radius of the feed spray region where the sprayed feed does not touch the inner wall of the column, which has the advantage of allowing a wider area of the feed spray region.

In an embodiment of the present disclosure, the spray nozzle 330 is provided in the inner pipe 320. Specifically, for the purpose of increasing the area of the feed spray region B relative to the number of inner pipes 320, a plurality of spray nozzles 330 may be individually provided in each of the plurality of inner pipes 320, and the plurality of spray nozzles may be spaced apart from each other.

For example, each of the plurality of inner pipes 320 may be individually provided with two or more, specifically two to four, and more specifically two spray nozzles 330 per one inner pipe. In this case, the plurality of spray nozzles 330 may be provided at the ends of each inner pipe 320. More specifically, the plurality of spray nozzles 330 may be provided at the ends and middle regions of each inner pipe, specifically, at a region between 1/3 and 2/3 of the total length of the inner pipe.

In an embodiment, the number of the spray nozzles may correspond to the number of the inner pipes in a ratio of 1:2. The total number of spray nozzles may be, for example, 4 to 16, specifically, 4 to 14, and more specifically, 4 to 12.

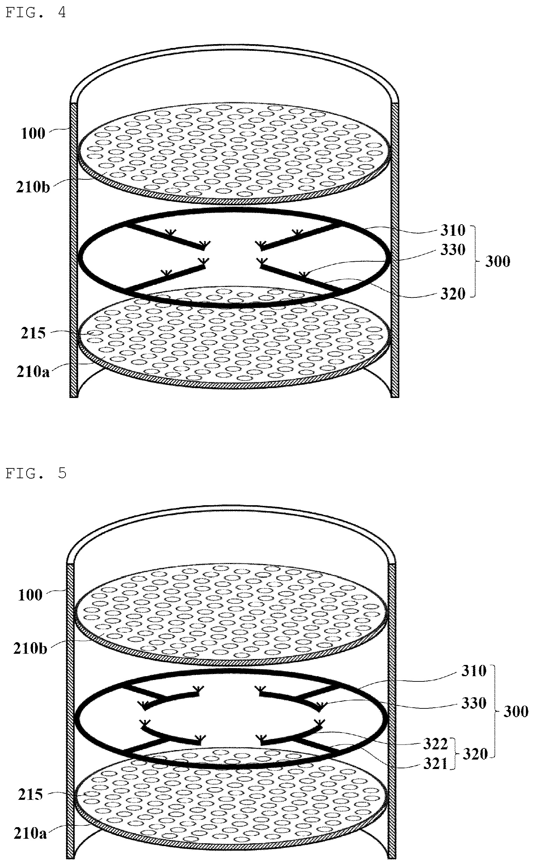

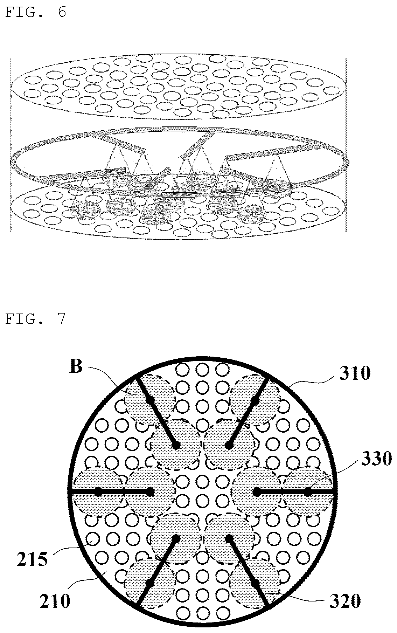

For example, in the case where the inner pipe 320 is the straight inner pipe, as illustrated in FIGS. 2 and 4, the spray nozzle 330 may be provided between the end of each inner pipe and the connection portion where the feed transfer pipe 310 and the inner pipe 320 are connected and the end of the inner pipe, that is, in a middle region of the inner pipe. In this case, the spray nozzle located in the middle region of the inner pipe should be spaced apart from the inner wall of the column so that the sprayed feed does not touch the inner wall of the column. For example, FIGS. 6 and 7 are examples in which six straight inner pipes are provided in the feed supply unit. Referring to FIGS. 6 and 7, it can be confirmed that the feed spray region B is widely and evenly distributed on the tray by providing two spray nozzles per inner pipe.

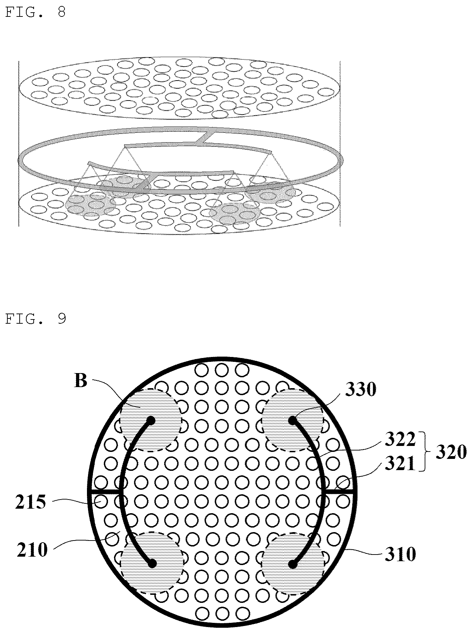

In addition, when the inner pipe 320 is the branched inner pipe, as illustrated in FIG. 3 and FIG. 5, the spray nozzle 330 may be provided at each of both ends of the branch pipe 322. For example, FIGS. 8 to 13 exemplarily illustrate feed spray regions B for each number of branched inner pipes.

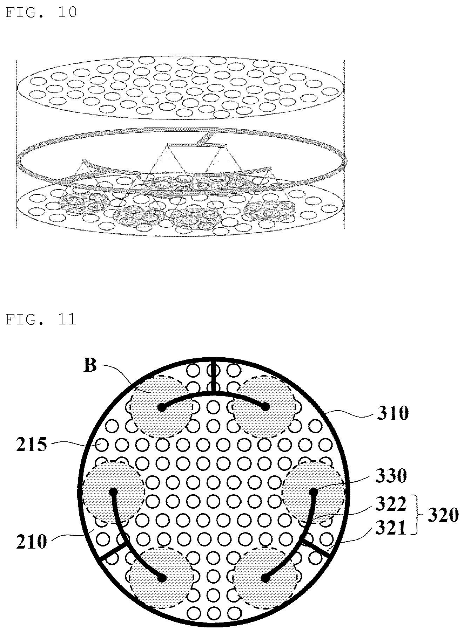

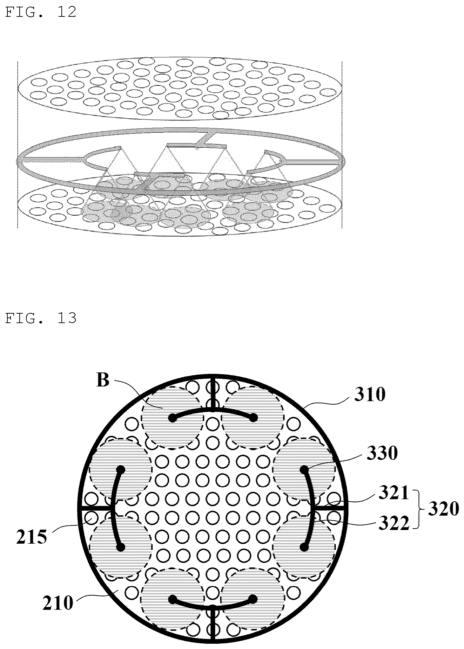

In addition, FIGS. 8 and 9 are examples of cases where two branched inner pipes are provided in the feed supply unit, FIGS. 10 and 11 are examples of cases where three branched inner pipes are provided in the feed supply unit, and FIGS. 12 and 13 are examples of cases where four branched inner pipes are provided in the feed supply unit. Referring to FIGS. 8 to 13, it can be confirmed that the feed spray region B is widely and evenly distributed on the tray by providing two or more spray nozzles 330 per one inner pipe 320.

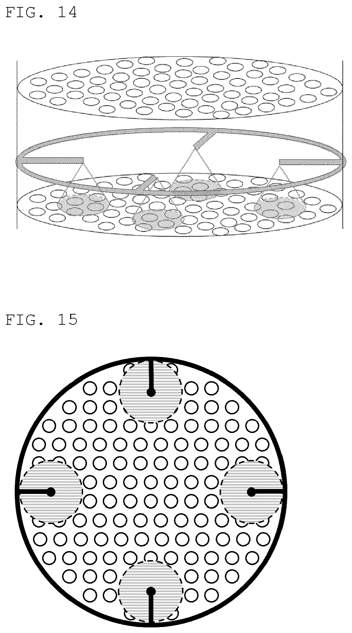

In order to compare the effect according to the structure of the feed supply unit 300, as a comparative example, the feed spray region B in the case where four inner pipes are provided as the feed supply unit, but only one spray nozzle is provided per inner pipe, is illustrated in FIGS. 14 and 15. Assuming that all conditions except the structure of the inner pipe and the number of spray nozzles per inner pipe are the same, when comparing the example of the present invention (FIGS. 12 and 13) in which the number of inner pipes is the same as four and the comparative example (FIGS. 14 and 15), it can be confirmed that the feed spray region B of the example is wider and more evenly distributed than that of the comparative example.

Meanwhile, the plurality of spray nozzles 330 may be arranged at the lower portion of the inner pipe 320 as illustrated in FIGS. 2 and 3, and may be provided to spray the feed toward the lower dual flow tray 210a. In addition, the plurality of spray nozzles 330 may be arranged at the upper portion of the inner pipe 320, as illustrated in FIGS. 4 and 5, and may be provided to spray the feed toward the upper dual flow tray 210b.

Specifically, the raw material sprayed from the plurality of spray nozzles 330 forms each feed spray region B in the lower dual flow tray 210a or the upper dual flow tray 210b depending on the direction in which the spray port of the spray nozzle 330 faces. In this case, it is desirable that each feed spray region B is formed with the largest possible area, but it is desirable that the feed spray regions do not overlap each other in order to uniformly supply the raw material. In addition, when the feed flows down the inner wall of the column 100, the separation efficiency is lowered because the gas-liquid contact is not made, and thus, the diameter of the feed spray region B should not exceed the inner diameter range of the cylindrical column 100.

Meanwhile, the height of the spray nozzle 330 can be determined according to the column inner diameter, the tray spacing TS, the spray angle X of the spray nozzle, the depth of the spray nozzle, and/or the feed spray direction, and can be the same as the height of the feed supply unit 300 described above. When the spray nozzle 330 is positioned too low, the spray nozzle 330 is positioned below the liquid level of the feed solution on the lower dual flow tray 210a, making it difficult to distribute the raw material over a wide area.

More specifically, in a case where the spray nozzle 330 is provided at the lower part of the inner pipe 320, when the spray nozzle 330 is positioned too high, the radius of the feed spray region B becomes excessively large, causing the feed to flow down along the inner wall of the column, which may lower the gas-liquid separation efficiency.

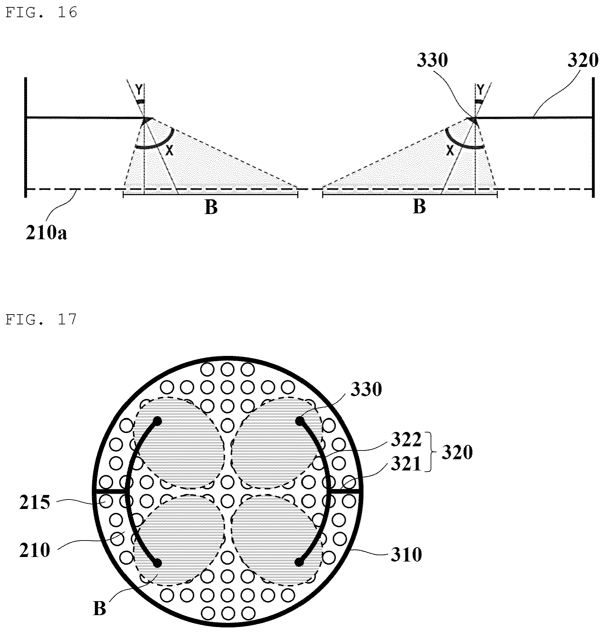

Meanwhile, referring to FIG. 16, based on (0°) when the spray nozzle is vertically installed in the upper or lower direction with respect to the inner pipe, each spray nozzle 330 may be installed at a predetermined angle Y in the direction of the central axis of the dual flow tray 210 to additionally expand the area of the feed spray region B. The angle adjustment of the spray nozzle may be more effective when applied in a case where the branched inner pipe is installed in terms of expanding the area of the feed spray region B and preventing overlapping between the feed spray regions B.

FIG. 16 and FIG. 17 are examples of a case where the feed supply unit is provided with the spray nozzle inclined at a predetermined angle Y, and illustrate the feed spray region B as an example. In general, in order to increase the area of the feed spray region B, the spray nozzle with a large spray angle X must be used, or the spray nozzle should be placed high. However, the spray angle X of the spray nozzle is fixed depending on the product, making it difficult to change, and there are limitations in terms of the product. In addition, there are limitations in adjusting the height of the spray nozzle due to the tray spacing. In this way, in cases where it is difficult to change conditions such as the spray angle X and height of the spray nozzle, as illustrated in FIG. 16, by adjusting the installation angle Y of the spray nozzle, as illustrated in FIG. 17, the area of the feed spray region B can be increased.

In addition, in the case of the upper direction spray as illustrated in FIGS. 4 and 5, when the distance between the spray nozzle 330 and the upper tray 210b is too long, the feed may not contact the upper tray and may fall in a parabolic manner. Therefore, when the spray nozzle is installed on the upper part of the inner pipe, the angle adjustment of the spray nozzle may be effective when the distance from the upper tray to the nozzle is smaller than the height of the spray nozzle.

More specifically, when the spray nozzle 330 is installed in a state of being tilted in the direction of the central axis, there is an advantage in that it is possible to prevent the feed from contacting the inner wall of the column even when the spray nozzle is shorter than the length of the inner pipe (or connecting pipe), the spray angle X is large, or the spray nozzle is installed high. However, when the feed sprayed from the spray nozzle 330 is sprayed parallel to the tray 210, the feed may be sprayed on the inner wall of the column opposite to the inner pipe where the spray nozzle is located, and thus, it is preferable that the installation angle Y of the spray nozzle satisfies the following relational expression 1.

[Relational Expression 1]

$$0.5X + Y < 90$$

In the relational expression 1, X is the spray angle of the spray nozzle, and Y is the angle of inclination based on the spray nozzle being installed vertically relative to the inner pipe (see FIG. 16).

More specifically, even when the feed is sprayed over an excessively large area compared to the inner diameter of the column, the feed may be sprayed on the inner wall of the column opposite the inner pipe where the spray nozzle is located, and thus, it is more preferable that the installation angle Y of the spray nozzle also satisfies the following relational expression 2.

[Relational Expression 2]

$$\tan(0.5X+Y) < (D-l)/h$$

In the relational expression 2, X is the spray angle of the spray nozzle, and Y is the angle of inclination based on the spray nozzle being installed vertically relative to the inner pipe (see FIG. 16), D is the inner diameter of the column, l is the depth of the spray nozzle, and h is the height of the spray nozzle.

For example, the theoretical calculation of the change in the area of the feed spray region according to the spray angle X of the spray nozzle and the installation angle Y of the spray nozzle is as illustrated in Table 1 below. Specifically, in Table 1 below, it is assumed that the area of the feed spray region is 1 when the spray angle X of the spray nozzle is 90° and the spray nozzle is installed in a vertical direction (Y=0°) with respect to the inner pipe so that the feed is sprayed, and the relative area ratio is calculated and organized according to the change in the spray angle X of the spray nozzle and the installation angle Y of the spray nozzle.

[Table 1]

| Installation angle Y | Area ratio of feed spray region (reference: area = 1 when X = 90°, Y = 0°) | | | | | |
|---|---|---|---|---|---|---|
| | X = 30° | X = 60° | X = 90° | X = 120° | X = 135° | X = 150° |
| 0° | 0.072 | 0.333 | 1.000 | 3.000 | 5.828 | 13.928 |
| 5° | 0.073 | 0.338 | 1.019 | 3.106 | 6.171 | 15.770 |
| 10° | 0.075 | 0.353 | 1.081 | 3.464 | 7.453 | 25.721 |
| 15° | 0.080 | 0.379 | 1.195 | 4.243 | 11.121 | - |
| 20° | 0.087 | 0.420 | 1.389 | 6.000 | - | - |
| 30° | 0.113 | 0.577 | 2.309 | - | - | - |
| 45° | 0.219 | 1.414 | - | - | - | - |
| 60° | 0.732 | - | - | - | - | - |

Referring to Table 1 above, for example, when the spray angle X of the spray nozzle is 30° and the installation angle Y of the spray nozzle is installed at 60°, the area of the feed spray region B theoretically increases by about 10 times, and when the spray angle X of the spray nozzle is 150° and the installation angle Y of the spray nozzle is installed at 10°, the area of the feed spray region B theoretically increases by about 1.85 times. Therefore, the smaller the spray angle X of the spray nozzle, the greater the effect of increasing the area of the feed spray region when the installation angle Y of the spray nozzle is adjusted, relatively.

Meanwhile, the separation device according to an embodiment of the present disclosure may further include a lower discharge port 400 provided at the bottom of the cylindrical column 100 to discharge a liquid stream, and an upper discharge port 500 provided at the top of the cylindrical column to discharge a gaseous stream.

The raw material supplied into the cylindrical column 100 undergoes a separation process through continuous gas-liquid contact at each stage of the column, and the relatively light low-boiling-point component rises in a vapor state and is discharged through the upper discharge port 500 provided at the top, and the relatively heavy high-boiling-point component descends in a condensed liquid state and can be discharged through the lower discharge port 400 provided at the bottom. For example, when a feed containing acrylic acid is supplied, the acrylic acid can be discharged through the lower discharge port 400, but is not limited thereto.

In the dual flow tray 210 of each stage, the gaseous stream rising upward and the liquid stream descending downward come into contact with each other to transfer heat and mass, and as a result, a portion of the high-boiling-point component is condensed and flows down to the bottom, and the uncondensed vapor continues to rise to the top, and the process is continuously performed.

As described above, the reactive monomer separation device according to the present disclosure is described and illustrated in the drawings, but the description and illustration of the drawings describe and illustrate only the core components for understanding the present disclosure, and in addition to the processes and devices described and illustrated in the drawings, processes and devices not described and illustrated separately can be appropriately applied and utilized to implement the reactive monomer separation device according to the present disclosure.

In the above, exemplary embodiments of the present disclosure have been described, but the present disclosure is not limited thereto, and those skilled in the art will understand that various changes and modifications are possible within the scope of the claims described below.

[Description of symbols]

| | | | |
|---|---|---|---|
| 100: | cylindrical column | 200: | stage |
| 210: | dual flow tray | 215: | hole |
| 300: | feed supply unit | 310: | feed transfer pipe |
| 320: | inner pipe | 330: | spray nozzle |
| 321: | connecting pipe | 322: | branch pipe |
| 400: | lower discharge port | 500: | upper discharge port |

**Claims**

1. A reactive monomer separation device, comprising:

   a cylindrical column;
   a plurality of dual flow trays provided inside the cylindrical column to partition a plurality of stages; and
   a feed supply unit provided in one of the plurality of stages to supply a raw material including a liquid reactive monomer,
   wherein the feed supply unit includes:

   a circular feed transfer pipe spaced apart between a lower dual flow tray and an upper dual flow tray of the stage in which the feed supply unit is provided and provided along an inner wall of the cylindrical column,
   a plurality of inner pipes extending in a direction of a central axis from the feed transfer pipe, and
   a plurality of spray nozzles provided in the plurality of inner pipes, and
   each of the plurality of inner pipes is individually provided with a plurality of the spray nozzles.

2. The reactive monomer separation device of claim 1, wherein the inner pipe includes:

   a connecting pipe extending from the feed transfer pipe in the direction of the central axis, and
   a branch pipe provided at an end of the connecting pipe.

3. The reactive monomer separation device of claim 2, wherein the branch pipe is provided in any one shape selected from a straight line and a curve.

4. The reactive monomer separation device of claim 2, wherein the spray nozzle is provided at each of both ends of the branch pipe.

5. The reactive monomer separation device of claim 1, wherein each of the spray nozzles is provided in a state of being inclined at a predetermined angle in the direction of the central axis.

6. The reactive monomer separation device of claim 1, wherein the plurality of spray nozzles are provided to spray a raw material toward the upper dual flow tray.

7. The reactive monomer separation device of claim 1, wherein the number of inner pipes is 2 to 8.

8. The reactive monomer separation device of claim 1, wherein the plurality of inner pipes are spaced apart from each other at equal intervals.

9. The reactive monomer separation device of claim 1, wherein the reactive monomer includes (meth)acrylic acid, acrylic acid, methyl acrylate, 1,3-butadiene, or a mixture thereof.

10. The reactive monomer separation device of claim 1, wherein the separation device is a reduced pressure distillation column.

11. The reactive monomer separation device of claim 1, further comprising:

a lower discharge port provided at a bottom of the cylindrical column for discharging a liquid stream; and
an upper discharge port provided at a top of the cylindrical column for discharging a gaseous stream.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

FIG. 15

FIG. 16

FIG. 17

# EP 4 691 590 A1

## INTERNATIONAL SEARCH REPORT

International application No.

**PCT/KR2024/008755**

| A. | CLASSIFICATION OF SUBJECT MATTER |
|---|---|

**B01D 3/32**(2006.01)i; **B01D 3/22**(2006.01)i; **B01D 3/42**(2006.01)i; **C07C 51/44**(2006.01)i; **C07C 67/54**(2006.01)i; **C07C 7/04**(2006.01)i; **C07C 57/04**(2006.01)i; **C07C 69/54**(2006.01)i; **C07C 11/167**(2006.01)i

According to International Patent Classification (IPC) or to both national classification and IPC

| B. | FIELDS SEARCHED |
|---|---|

Minimum documentation searched (classification system followed by classification symbols)

B01D 3/32(2006.01); B01D 3/00(2006.01); B01D 53/14(2006.01); B01D 53/48(2006.01); B01J 4/00(2006.01); B08B 9/093(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 듀얼플로우트레이(dual flow tray), 증류(distillation), 장치(apparatus), 노즐(nozzle), 배관(pipe), 모노머(monomer)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | US 2020-0179889 A1 (ARKEMA FRANCE) 11 June 2020 (2020-06-11)<br>See claims 1, 5 and 8. | 1-11 |
| A | CN 108025220 A (BASF SE) 11 May 2018 (2018-05-11)<br>See claims 1 and 2. | 1-11 |
| A | KR 10-2009-0017131 A (AMTPACIFIC CO., LTD.) 18 February 2009 (2009-02-18)<br>See claim 1. | 1-11 |
| A | US 2004-0249198 A1 (THIEL, Joachim et al.) 09 December 2004 (2004-12-09)<br>See claims 11 and 20. | 1-11 |
| A | KR 10-2002-0011774 A (ACTIMAG CO. et al.) 09 February 2002 (2002-02-09)<br>See claim 1. | 1-11 |

☐ Further documents are listed in the continuation of Box C.    ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **25 September 2024** | **26 September 2024** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
Information on patent family members

International application No.

**PCT/KR2024/008755**

| Patent document cited in search report | | | Publication date (day/month/year) | Patent family member(s) | | | Publication date (day/month/year) |
|---|---|---|---|---|---|---|---|
| US | 2020-0179889 | A1 | 11 June 2020 | BR | 112016000817 | A2 | 25 July 2017 |
| | | | | BR | 112016000817 | B1 | 09 November 2021 |
| | | | | CN | 105377412 | A | 02 March 2016 |
| | | | | CN | 105377412 | B | 04 December 2018 |
| | | | | EP | 3024564 | A1 | 01 June 2016 |
| | | | | EP | 3024564 | B1 | 08 July 2020 |
| | | | | FR | 3008899 | A1 | 30 January 2015 |
| | | | | FR | 3008899 | B1 | 30 January 2015 |
| | | | | JP | 2016-527240 | A | 08 September 2016 |
| | | | | JP | 6827807 | B2 | 10 February 2021 |
| | | | | KR | 10-2016-0034935 | A | 30 March 2016 |
| | | | | KR | 10-2187726 | B1 | 07 December 2020 |
| | | | | MX | 2016000771 | A | 27 April 2016 |
| | | | | SG | 11201600553 | A | 26 February 2016 |
| | | | | US | 10596537 | B2 | 24 March 2020 |
| | | | | US | 10919015 | B2 | 16 February 2021 |
| | | | | US | 2016-0175796 | A1 | 23 June 2016 |
| | | | | WO | 2015-011048 | A1 | 29 January 2015 |
| | | | | ZA | 201508870 | B | 29 November 2017 |
| CN | 108025220 | A | 11 May 2018 | BR | 112018000793 | A2 | 04 September 2018 |
| | | | | BR | 112018000793 | B1 | 03 March 2022 |
| | | | | CN | 108025220 | B | 30 March 2021 |
| | | | | DE | 102015213493 | A1 | 15 September 2016 |
| | | | | EP | 3325122 | A1 | 30 May 2018 |
| | | | | EP | 3325122 | B1 | 05 February 2020 |
| | | | | US | 2017-0014730 | A1 | 19 January 2017 |
| | | | | WO | 2017-012855 | A1 | 26 January 2017 |
| KR | 10-2009-0017131 | A | 18 February 2009 | None | | | |
| US | 2004-0249198 | A1 | 09 December 2004 | DE | 10156988 | A1 | 28 May 2003 |
| | | | | DE | 10295317 | B4 | 28 March 2013 |
| | | | | DE | 10295317 | D2 | 09 September 2004 |
| | | | | JP | 2005-509512 | A | 14 April 2005 |
| | | | | JP | 2005-509512 | T | 14 April 2005 |
| | | | | JP | 4116558 | B2 | 09 July 2008 |
| | | | | US | 7306204 | B2 | 11 December 2007 |
| | | | | WO | 03-043712 | A1 | 30 May 2003 |
| | | | | WO | 0304-3712 | A8 | 07 August 2003 |
| KR | 10-2002-0011774 | A | 09 February 2002 | KR | 10-0375605 | B1 | 10 March 2003 |

Form PCT/ISA/210 (patent family annex) (July 2022)

**REFERENCES CITED IN THE DESCRIPTION**

**Patent documents cited in the description**

- KR 1020230124667